# EUROPÄISCHE PATENTSCHRIFT

(11) **EP 1 727 539 B1**
(45) Veröffentlichungstag und Bekanntmachung des Hinweises auf die Patenterteilung: **31.10.2007**
(21) Anmeldenummer: 05728686.6
(22) Anmeldetag: 22.03.2005
(51) Int. Cl.: A61K 31/381, A61P 25/16

(54) **VERWENDUNG VON ROTIGOTIN ZUR BEHANDLUNG UND PRÄVENTION DES PARKINSON-PLUS-SYNDROMS**
USE OF ROTIGOTINE FOR TREATING AND PREVENTING PARKINSON'S PLUS SYNDROME
UTILISATION DE ROTIGOTINE POUR TRAITER ET PREVENIR LE SYNDROME PARKINSON PLUS

(30) Priorität: 24.03.2004 DE 102004014841
(43) Veröffentlichungstag der Anmeldung: 06.12.2006
(73) Patentinhaber: SCHWARZ PHARMA AG, 40789 Monheim/Rhld. (DE)
(72) Erfinder: SCHELLER, Dieter, 41470 Neuss (DE)
(74) Vertreter: HOFFMANN EITLE
(86) Internationale Anmeldenummer: PCT/EP2005/003013
(87) Internationale Veröffentlichungsnummer: WO 2005/092331

(56) Entgegenhaltungen:
- WO-A-96/31210
- WO-A-20/05070428
- TUITE P ET AL: "RECENT DEVELOPMENTS IN THE PHARMACOLOGICAL TREATMENT OF PARKINSON'S DISEASE" EXPERT OPINION ON INVESTIGATIONAL DRUGS, ASHLEY PUBLICATIONS LTD., LONDON, GB, Bd. 12, Nr. 8, 1. August 2003 (2003-08-01), Seiten 1335-1352, XP009023949 ISSN: 1354-3784
- PASCUAL J ET AL: "DOPAMINE D1 AND D2 RECEPTORS IN PROGRESSIVE SUPRANUCLEAR PALSY: AN AUTORADIOGRAPHIC STUDY" ANNALS OF NEUROLOGY, BOSTON, US, Bd. 32, Nr. 5, 1992, Seiten 703-707, XP008035931 ISSN: 0364-5134
- PIEROT L ET AL: "D1 AND D2-TYPE DOPAMINE RECEPTORS IN PATIENTS WITH PARKINSON'S DISEASE AND PROGRESSIVE SUPRANUCLEAR PALSY" JOURNAL OF NEUROLOGICAL SCIENCES, ELSEVIER SCIENTIFIC PUBLISHING CO, AMSTERDAM, NL, Bd. 86, 1988, Seiten 291-306, XP008035928 ISSN: 0022-510X
- DAAS DEN I ET AL: "IMPROVEMENT OF THE ORAL BIOAVAILABILITY OF THE SELECTIVE DOPAMINE AGONIST N-0437 IN RATS: THE IN VITRO AND IN VIVO ACTIVITY OF EIGHT ESTER PRODRUGS" NAUNYN-SCHMIEDEBERG'S ARCHIVES OF PHARMACOLOGY, SPRINGER, BERLIN, DE, Bd. 341, 1990, Seiten 186-191, XP009046188 ISSN: 0028-1298
- NEOPHYTIDES A., LIEBERMAN A.: "The use of lisuride, a potent dopamine and serotonine agonist in the treatment of progressive supranuclear palsy" J. NEUROL. NEUROSURG. PSYCH., Bd. 45, 1982, Seiten 261-263, XP008052053

## Beschreibung

Unter dem Begriff Parkinson-Plus-Syndrom werden einige idiopathische Krankheiten zusammengefasst, die mit dem Auftreten von Parkinson-ähnlichen Symptomen verbunden sind, die sich jedoch diagnostisch und klinisch/pathophysiologisch von Morbus Parkinson abgrenzen lassen.

Dem Krankheitskomplex der Parkinson-Plus-Syndrome (PPS) werden die Multisystematrophien (MSA), die progressive supranucleäre Blickparese (PSP), die kortico-basalganlionäre Degeneration (CBGD) und die Demenz mit Lewy Bodies (DLB) zugeordnet.

Unter Multisystematrophien sind insbesondere das Sky-Drager-Syndrom, die Olivo-ponto-zerebelläre Atrophie (OPCA) und die Striatonigrale Degeneration (SND) zu subsumieren (Mark et al, Neurol Clin. 2001, 19(3): 607).

Die Zuordnung der Pick'schen Krankheit, des Hemiparkinsonismus und des Parkinsonismus bei Alzheimer- und ALS-Patienten sowie die Westphal-Variante von Chorea Huntington zu den PPS ist in der Fachliteratur nicht einheitlich, doch sollen diese Erkrankungen in der vorliegenden Patentanmeldung entsprechend der Einteilung von Hobson et al. unter dem Begriff PPS subsumiert werden (Hobson et al, Can J Neurol Sci. 2003 Mar; 30 Suppl 1: S2).

Den unter Parkinson-Plus-Syndrom subsumierten Erkrankungen ist das fehlende oder rasch nachlassende Ansprechen auf L-Dopa bzw. Dopamin-Agonisten sowie zusätzliche Symptome wie zerebellare oder Pyramidenbahnzeichen, frühe oder schwere Demenz sowie Sprech- und Schluckstörung in der Frühphase gemein (Mark 2001, supra; Gerlach et al, Die Parkinson-Krankheit, Springer, Wien New York, 2003).

Eine Übersicht über differential-diagnostische Kriterien einiger Parkinson-Plus Syndrome und Morbus Parkinson (auch als idiopathisches Parkinson Syndrom, IPS, bezeichnet) gibt Tabelle 1.

**Tabelle 1: Übersicht über die Klassifizierung einiger Krankheitssymptome parkinsonähnlicher Bewegungsstörungen und differentialdiagnostische Charakteristika**

| | **Parkinson-Plus-Syndrom** | | | | **IPS** |
|---|---|---|---|---|---|
| | **Multisystematrophie** | | **KBD** | **PSP** | |
| | **SND** | **OPCA** | | | |
| Rigor /Akinese | ++ | + | ++/+++ | ++/+++ | ++/+++ |
| Zerebellare Zeichen | + | ++ | - | - | - |
| Pyramidenbahnzeichen | - | ++ | ++ | + | - |
| Posturale Instabilität | + | + | + | +++ | + |
| Demenz | - | - | + | + | + |
| Okulomotorische Störungen | + | (+) | + | +++ | + |
| Dysphagie | - | + | ++ | ++ | + |
| Retrocollies | - | - | - | ++ | - |
| Sphinkter-Störungen | + | + | - | - | - |
| Impotenz | + | + | ++ | + | + |

| | | | | | |
|---|---|---|---|---|---|
| SND: striato-nigrale Degeneration OPCA: olivo-ponto-zerebelläre Atrophie KBD: kortiko-basale Degeneration PSP: progressive supranukleäre Blick-Lähmung IPS: idiopathisches Parkinson-Syndrom (modifziert nach Mark MH, Lumping and splitting the Parkinson Plus syndromes: dementia with Lewy bodies, multiple system atrophy, progressive supranuclear palsy, and cortical-basal ganglionic degeneration. Neurol Clin. 2001 Aug;19(3):607-27 und Gerlach M, Reichmann H, Riederer P, Die Parkinson-Krankheit, Springer Wien New York, 2003). | | | | | |

Ein wichtiges Unterscheidungskriterium zwischen IPS und PPS stellt die Computertomographie dar. Patienten mit IPS weisen im SPECT bis auf die Spätstadieh einen normalen Dopamin-Rezeptorenbesatz auf; PPS Patienten hingegen weisen einen frühen Verlust prä- und postsynpatischer dopaminerger Neurone auf, was mit einer nachweislichen Reduktion der Dichte der Dopamin-Rezeptoren einhergeht. PET-Analysen belegen einen verminderten L-DOPA-Gehalt und L-DOPA-Metabolismus in IPS Patienten (Gerlach et al., 2003, supra).

Die medikamentöse Therapie von PPS ist auf Grund des häufig fehlenden oder schlechten Ansprechens von L-Dopa schwierig und besteht im Allgemeinen in einer symptomatischen Therapie bestimmter Einzelsymptome, z.B. der Therapie der Hypotension.

Dopaminrezeptor-Agonisten sind bei der Therapie von PPS in der Regel ineffektiv (Mark, 2001, supra). In Ausnahmefällen wurden gewisse Therapieerfolge mit einzelnen Dopaminagonisten berichtet, doch scheinen diese Effekte substanzspezifisch zu sein. Beispielsweise berichten Wenning et al (Lancet, 2004, 3, 93) von Therapieerfolgen von Bromocriptin in einer Studie mit sechs Patienten, wohingegen eine kontrollierte Studie mit Lisurid keine Effektivität zeigte. Da bekannt ist, dass die meisten Dopaminagonisten nicht nur auf einen Dopaminrezeptor einwirken, sondern ein komplexes Rezeptorprofil aufweisen (Newman-Tancredi, J Pharmacol Exp Ther 2002, 303, 805), kann die Ursache für die Wirksamkeit von Bromocriptin in den Besonderheiten des Rezeptorprofils oder anderen nicht weiter charakterisierten substanzspezifischen Eigenschaften begründet sein.

Das Parkinson-Plus-Syndrom ist in der Regel mit einer dopaminergen Neurodegeneration in der Substantia nigra verbunden (Mark, 2001, supra). Eine erfolgreiche Therapie darf daher vom Einsatz wirksamer Neuroprotektiva erwartet werden, die den fortschreitenden Abbau dopaminerger Neuronen hemmen (Dawson and Dawson VL, Nat Neurosci. 2002 Nov: 5 Suppl:1.058).

Rotigotin [(-)-5,6,7,8-Tetrahydro-6-[propyl[2-(2-thienyl)ethyl]amino]-1-naphtol] ist aus dem Stand der Technik als Dopaminagonist und symptomatisches Morbus Parkinson-Therapeutikum bekannt.

Die WO 02/089777 beschreibt beispielsweise die transdermale Gabe von Rotigotin bei Parkinson-Patienten und die damit verbundene Verbesserung des UPDRS (Unified ' Parkinson's Disease Rating Scale)-Scores wie auch verschiedene andere Autoren (Metman et al, Clin Neuropharmacol. 2001,24(3):163; Mucke HA, Rotigotin Schwarz Pharma. IDrugs. 2003 Sep; 6(9):894; The Parkinson Study Group, Arch Neurol. 2003 60(12):1721).

Der UPDRS-Score ist ein wichtiges Instrument zur Diagnose bzw. der Therapie von Morbus Parkinson-Patienten (Fahn S, Elton RL, Members of the UPDRS Development Committee (1987) Unified Parkinson's Disease Rating Scale. In: Fahn, S, CD Marsden, DB Calne, M Goldstein (eds) Recent Devlopments in Parkinson's Disease. Vol 11. Macmillan Healthcare Information, Florham Park (NJ), pp 153-163, 293-304). Allerdings wird mit dem UPDRS-Score lediglich der Effekt eines Wirkstoffs auf die Morbus Parkinson-Symptomatik erfasst. Er erlaubt keine direkte Aussage darüber, ob ein Wirkstoff den der Symptomatik zugrunde liegenden dopaminergen Zelluntergang beeinflusst.

Apoptotische Vorgänge spielen aber insbesondere beim Untergang dopaminerger Neurone in der Pathogenese von Parkinson-Plus eine wichtige Rolle (Lev et al, Prog Neuropsychopharmacol Biol Psychiatry. 2003;27(2):245; Michel et al, Rev Neurol (Paris). 2002;158 Spec no 1: S24). Des Weiteren sollen verschiedene andere neurodegenerative Prozesse die Entstehung des Parkinsonismus und des Parkinson-Plus maßgeblich beeinflussen (Hirsch et al, Ann N Y Acad Sci. 2003; 991: 214).

Es werden daher neuroprotektive Substanzen gewünscht, die den dopaminergen Zelluntergang stoppen oder sogar umkehren können (Vila et al, Nat Rev Neurosci. 2003; 4(5): 365). Als prädiktiv für die geforderten neuroprotektiven Eigenschaften gilt dabei das MTPT-Modell (Dawson, 2002, supra; Eberhardt O, Schulz JB, Toxicol Lett. 2003, 139(2-3):135).

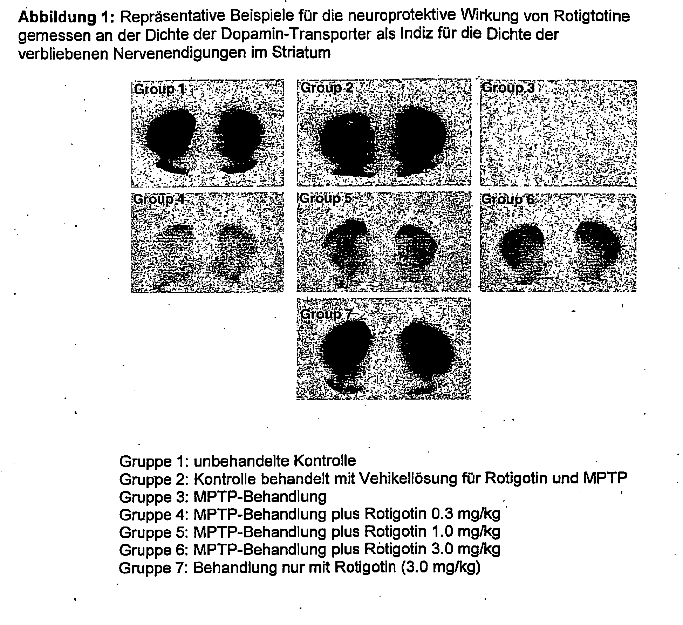

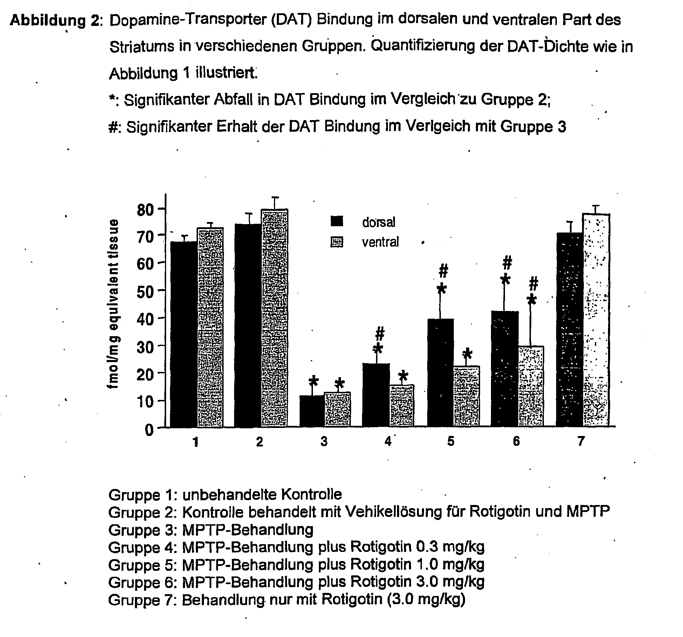

Experimentelle Untersuchungen zeigten nun überraschend, dass das bisher nur zur symptomatischen Therapie der idiopathischen Parkinson'schen Krankheit eingesetzte Rotigotin neuroprotektive Eigenschaften besitzt. Rotigotin zeigt sowohl in einem akuten wie in einem subakuten MTPT-Modell überraschend das gewünschte pharmakologische Profil (Tabelle 2, Abbildungen 1 und 2). Die Untersuchungsergebnisse legen nahe, dass mit Rotigotin apoptotische Prozesse verhindert werden.

Rotigotin zeigt beispielsweise neuroprotektive Wirkung in einem Parkinsonmodell der Maus: Nach akuter Gabe von MPTP, das bei Menschen wie bei Affen Parkinson-Symptome erzeugt, wurde zum einen die Anzahl der in der akuten Phase degenerierenden Neurone gemessen und zum anderen die funktionelle Integrität des Striatums in der subaktuen Phase durch Bestimmung der Dichte des Dopamin-Transporters in den terminalen Nervenendigungen erfasst. In beiden Fällen konnte gezeigt werden, dass Rotigotin neuroprotektiv wirksam war: Zum einen war die Anzahl degenerierender Neurone im Mesenzephalon nach der Gabe von Rotigotin verringert (Tabelle 2) und zum anderen war die dopaminerge Innervation des Striatums nahezu vollständig erhalten bzw. wieder hergestellt (Abbildungen 1 und 2).

**Tabelle 2: Anzahl akut degenerierender Neurone im MPTP-Maus-Modell dargestellt mit FluoroJade Färbung mit und ohne Behandlung mit einer Einmalgabe von Rotigotin**

| Gruppe | Anzahl degen. Neurone | Stndrtabw |
|---|---|---|
| 1: Vehikel-behandelte Kontrolle | 2.0 | 2.4 |
| 2: MPTP Intoxikation | 73.5 | 34.0 |
| 3: MPTP Intoxikation + Rotigotin 0.3 mg/kg | 66.7 | 30.5 |
| 4: MPTP Intoxikation + Rotigotin 1.0 mg/kg | 76.8 | 41.6 |
| 5: MPTP Intoxikation + Rotigotin 3.0 mg/kg | 34.9 | 31.9 |
| 5: MPTP -Vehikel + Rotigotin 3.0 mg/kg | 3.8 | 4.3 |

In einer Pilotstudie wurde auch die neuroprotektive Wirkung von Rotigotin an Affen untersucht.

In dem verwendeten Modell, das den progressiven Verlauf des dopaminergen Zelluntergangs an Primaten widerspiegelt; wurden Affen (Makaken) unterschwelligtoxische Dosen von MPTP über mehrere Tage injiziert. Die Parkinsonismus-Symptomatik entwickelte sich in dem Modell über einen Zeitraum von ca 2 Wochen. Sobald ein bestimmter Grad der Schädigung erreicht war, wurden tägliche Injektionen von Rotigotin in einer Formulierung vorgenommen, die einen kontinuierlichen Plasmaspiegel über 24 h erzeugt..Die Injektionen von MPTP wurden gestoppt, sobald die motorische Aktivität in den Kontrollen um einen bestimmten Grad vermindert war (ca 5 Tage später). Das Verhalten der Tiere wurde täglich bewertet. Sechs Wochen nach Beginn der MPTP-Applikation wurden die Injektionen von Rotigotin gestoppt und die Tiere wurden für weitere zwei Wochen ohne Behandlung beobachtet. Es wurde beobachtet, daß die motorische Aktivität der Tiere unter Behandlung und auch in der folgenden Auswaschphase deutlich verbessert war.

Am Ende der Rotigotin-Applikation bzw. am Ende der Auswaschphase wurde je eine Gruppe von Tieren getötet und der Zustand der Basalganglien histologisch und biochemisch untersucht. Die Dichte der Nervenendigungen im Striatum war gegenüber den unbehandelten Tieren deutlich erhöht. Der Gehalt an Pre-Pro-Enkephalin, ein Indikator für die intakte Vernetzung im 'indirect pathway' der Basalganglien, zeigte nach der Behandlung und nach der Auswaschphase eine Tendenz zur Normalisierung.

Die Resultate zeigen, daß das neuroprotektive Potential von Rotigotin auch in einem Primatenmodel des dopaminergen Zelluntergangs nachgewiesen werden kann. Damit kann eine antiapoptotische, neuroprotektive Wirkung auch beim Menschen angenommen werden.

Mit Rotigotin wurde damit der Therapie ein Wirkstoff zur Verfügung gestellt, der in idealer Weise zur Herstellung eines Arzneimittels, bzw. Prophylaktikums zur Behandlung und/oder zur Prävention des dopaminergen Neuronenverlustes in Patienten mit Parkinson-Plus-Syndrom geeignet ist, da sich neben der neuroprotektiven Wirkung auch die dopaminerge Wirkung des Rotigotins vorteilhaft auswirken kann.

Im Vergleich zur bisherigen Anwendung von Rotigotin, die auf die rein symptomatische, dopaminerge Behandlung von Morbus Parkinson-Patienten beschränkt war, wird damit als neues Anwendungsgebiet die Behandlung von Patienten mit Parkinson-Plus-Syndrom erschlossen, und zwar auch von solchen Patienten, die nicht oder unzureichend auf die Behandlung mit L-Dopa oder Dopaminagonisten ohne neuroprotektive Wirkung ansprechen.

Ein Gegenstand der Erfindung ist daher die Verwendung von Rotigotin, seiner Salze und Prodrugs als Arzneimittel zur Prävention und/oder Behandlung des Parkinson-Plus-Syndroms, wobei der Begriff des Parkinson-Plus-Syndroms die folgenden Erkrankungen umfasst: Multisystematrophien, progressive supranukleäre Blickparese, kortikobasale Degeneration, Demenz mit Lewy-Körperchen, Pick'sche Krankheit, Hemiparkinsonismus, Parkinsonismus bei Alzheimer- und ALS-Patienten sowie die Westphal-Variante von Chorea Huntington. Die mit Rotigotin zu behandelnden Erkrankungen sind dabei bevorzugt ausgewählt aus der Gruppe der Multisystematrophien, der progressiven supranukleären Blickparese, der kortikobasalen Degeneration und der diffusen Lewy-Körperchen-Demenz.

Ein anderer Gegenstand der Erfindung ist eine Methode zur Behandlung eines Patienten mit Parkinson-Plus Syndrom durch Verabreichung einer therapeutisch ausreichenden Menge von Rotigotin, seiner Salze und/oder Prodrugs, bzw. durch Verabreichung eines Arzneimittels, das Rotigotin oder eines seiner Salze und/oder Prodrugs nach Anspruch 1 enthält.

Unter "Prodrugs" von Rotigotin werden in dieser Patentanmeldung Verbindungen verstanden, die im menschlichen Körper, insbesondere im Plasma oder beim Durchtritt durch Haut oder Schleimhaut in therapeutisch effektiver Menge zu Rotigotin gespalten, umgesetzt oder metabolisiert werden, wie in folgendem Absatz erklärt.

Rotigotin hat die Formel

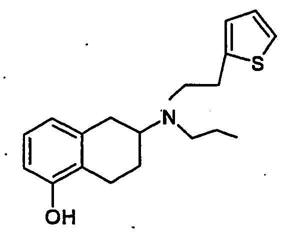

Als Prodrugs von Rotigotin kommen daher Derivate der phenolischen Hydroxygruppe in Frage, nämlich Ester, d.h Arylcarbonylester, Alkylcarbonylester oder Cycloalkylcarbonylester, insbesondere Alkylcarbonylester und Cycloalkylcarbonylester mit jeweils bis zu 6 Kohlenstoffatomen; Carbonate; Carbamate; Acetale; Ketale; Acyloxyalkylether; Phosphate; Phosphonate; Sulfate; Sulfonate; Thiocarbonylester; Öxythiocarbonylester; Thiocarbamate; Ether und Silylether.

Der Begriff "Alkylcarbonylester" umfasst Verbindungen, in denen jeweils das Sauerstoffatom des Rotigotins an die Gruppe -C(O)-Alkyl gebunden ist. Ein Alkylcarbonylester entsteht formal aus der Veresterung der phenolischen Hydroxygruppe mit einer Alkansäure, z.B. mit Essigsäure, Propionsäure, Buttersäure, Isobuttersäure oder Valeriansäure.

Der Begriff "Cycloalkylcarbonyleste" umfasst Verbindungen, in denen jeweils das Sauerstoffatom des Rotigotins an die Gruppe -C(O)-Cycloalkyl gebunden ist.

Der Begriff "Arylcarbonylester" umfasst Verbindungen, in denen jeweils das Sauerstoffatom des Rotigotins an die Gruppe -C(O)-Aryl gebunden ist.

Der Begriff "Carbonate" umfasst Verbindungen, in denen jeweils das Sauerstoffatom des Rotigotins an die Gruppe -C(O)-O-R gebunden ist.

Der Begriff "Carbamat" umfasst Verbindungen, in denen jeweils das Sauerstoffatom des Rotigotins an die Gruppe -C(O)-NRR1, -C(O)-NH-R1 oder-C(O)-NH₂ gebunden ist.

Der Begriff "Acetal" umfasst Verbindungen, in denen jeweils das Sauerstoffatom des Rotigotins an die Gruppe -CH(OR)R1 gebunden.ist.

Der Begriff "Keb31" umfasst Verbindungen, in denen jeweils das Sauerstoffatom des Rotigotins an die Gruppe -C(OR)R1 R2 gebunden ist.

Der Begriff "Acyloxyalkylether" umfasst Verbindungen, in denen jeweils das Sauerstoffatom des Rotigotins an die Gruppe -CHR-O-C(O)-R1 oder CH₂-O-C(O)-R1 gebunden ist.

Der Begriff "Phosphat" umfasst Verbindungen, in denen jeweils das Sauerstoffatom des Rotigotins an die Gruppe -P(O₂H)OR gebunden ist.

Der Begriff "Phosphonat" umfasst Verbindungen, in denen jeweils das Sauerstoffatom des Rotigotins an die Gruppe -P(O₂H)R gebunden ist.

Der Begriff "Sulfat" umfasst Verbindungen, in denen jeweils das Sauerstoffatom des Rotigotins an die Gruppe-S(O)₂OR gebunden ist.

Der Begriff "Sulfonat" umfasst Verbindungen, in denen jeweils das Sauerstoffatom des Rotigotins an die Gruppe-S(O)₂R gebunden ist.

Der Begriff "Thiocarbonylester" umfasst Verbindungen, in denen jeweils das Sauerstoffatom des Rotigotins an die Gruppe -C(=S)-R gebunden ist.

Der Begriff "Oxythiocarbonylester" umfasst Verbindungen, in denen jeweils das Sauerstoffatom des Rotigotins an die Gruppe -C(=S)-O-R gebunden ist.

Der Begriff "Thiocarbamat" umfasst Verbindungen, in denen jeweils das Sauerstoffatom des Rotigotins an die Gruppe -C(=S)-N-RR1,-C(=S)-NH-R1 oder -C(=S)-NH₂ gebunden ist.

Der Begriff "Ether" umfasst Verbindungen, in denen jeweils das Sauerstoffatom des Rotigotins an die Gruppe -R gebunden ist.

In den obigen Definitionen der Prodrugs sind R, R1, R2 jeweils unabhängig ausgewählt aus Wasserstoff, Alkyl, Cycloalkyl oder Aryl, und bevorzugt aus der Gruppe C1-6 Alkyl, C3-10 Cycloalkyl, und Phenyl.

"Alkyl" kann eine verzweigte oder unverzweigte Alkylgruppe sein, die vorzugsweise 1 bis 10 C-Atome, besonders bevorzugt 1 bis 6 C-Atome aufweist. Alkylgruppen können zusätzlich mit einem oder mehreren Substituenten substituiert sein, beispielsweise mit. Halogen.

"Cycloalkyl" ist eine Alkylgruppe, die nur aus reinen ringbildenden C-Atomen bestehen kann oder optional weitere verzweigende C-Atome tragen kann. Bevorzugte Kettenlängen sind 3-10, besonders bevorzugt 4-8 oder 4-6 C-Atome.

"Aryl" ist bevorzugt Phenyl. Phenyl kann gegebenenfalls zusätzlich in einer oder mehreren Positionen substituiert sein, z.B. mit Alkoxy, Alkyl, Halogen oder Nitro.

Die Herstellung durch Reaktion von Rotigotin mit entsprechenden reaktiven Vorstufen wie Säurechloriden, Säureanhydriden, Carbamylchloriden, Sulfonylchloriden etc. ist dem Fachmann auf dem Gebiet der klinischen Chemie bekannt und lassen sich der einschlägigen Fachliteratur entnehmen. Beispiele für Literaturstellen sind Bundgaard: Design of Prodrugs, Elsevier, Amsterdam, 1985; Higuchi und Stella: Pro-drugs as novel drug delivery systems in American Chemical Society, Washington DC, 1975; Sloan: Prodrugs - Topical and Ocular Drug Delivery, Ed: M. Dekker, 1992; Roche: Design of biopharmaceutical properties through prodrugs and analogs, Washington, DC, 1977.

Verschiedene Prodrugs des Razemats von Rotigotin (N-0437) sowie entsprechende Herstellvorschriften und Untersuchungsmethoden sind beispielsweise beschrieben in Den. Haas et al, Naunyn-Schmiedeberg's Arch Pharmacol 1990, 342. 655; Den Haas et al, Naunyn Schmiedebergs Arch Pharmacol. 1990, 341. 186 und Den Haas et al, J Pharm Pharmacol 1991, 43. 11.

Die grundsätzliche Eignung eines Rotigotin-Derivats als Prodrug kann bestimmt werden, indem die jeweilige Verbindung unter definierten Bedingungen mit einem Enzymcocktail, einem Zellhomogenisat oder einer enzymhaltigen Zellfraktion inkubiert wird und nachgewiesen wird, dass Rotigotin in ausreichendem Maße gebildet werden kann. Eine geeignete Enzymmischung ist beispielsweise enthalten in der S 9-Leberpräparation der Firma Gentest, Woburn, Ma, USA (Ausführungsbeispiel 5). Alternativ kann eine Inkubation mit Frischblut oder Plasma oder aber eines Homogenates der Unterhaut erfolgen, um eine leberunabhängige Metabolisierung der Prodrugs zur Wirkkomponente zu demonstrieren. Für transdermale Applikation ist eine in vitro Untersuchung der Permeation an exzidierter Haut notwendig.

Der endgültige Nachweis der Eignung und potentiellen Wirksamkeit in den Krankheitsmodellen erfolgt durch eine Bestimmung des aus dem Prodrug gebildeten Rotigotins im Plasma: In-vivo sollte ein Prodrug soviel Rotigotin freisetzen, dass eine therapeutisch effektive steady-state Konzentration Rotigotin im Plasma erreicht wird wie sie aus klinischen oder praeklinischen Untersuchungen bereits bekannt ist. Als effektive Konzentrationen werden dabei im allgemeinen Rotigotinkonzentrationen zwischen 0.01 und 50 ng/mL, bevorzugt zwischen 0.05 ng und 20 ng/mL und besonders bevorzugt zwischen 0.1 und 10 ng/mL Plasma angesehen.

Rotigotin ist das S(-)-Enantiomer von 5,6,7,8-Tetrahydro-6-[propyl[2-(2-thienyl)ethyl]amino-1-naphtol. Dies bedeutet, dass der Anteil des (R)-Enantiomeren im Arzneimittel erfindungsgemäß gering ist. Das (R)-Enantiomer liegt bevorzugt mit einem Anteil von < 10 Mol%, besonders bevorzugt mit einem Anteil von < 2 Mol% und ganz besonders bevorzugt mit einem Molanteil von < 1 %, bezogen auf die Gesamtmenge Rotigotin, im Arzneimittel vor.

Rotigotin und seine Prodrugs können als freie Basen oder in Form der physiologisch akzeptablen Salze, z.B. in Form des Hydrochlorids, im Arzneimittel vorliegen.

"Physiologisch akzeptable Salze" schließen nicht-toxische Additionssalze von Rotigotin mit organischen oder anorganischen Säuren, z.B. HCl, ein.

Zur Verabreichung von Rotigotin und seinen Prodrugs stehen verschiedene Applikationswege zur Verfügung, die der Fachmann je nach Bedarf, Zustand und Alter des Patienten, erforderlicher Dosierung und gewünschtem Applikationsintervall auswählen und anpassen kann.

Eine bevorzugte Art der Verabreichung von Rotigotin ist die transdermale Gabe. Die Darreichungsform kann grundsätzlich ausgewählt sein aus z.B. Salbe, Paste, Spray, Folie, Pflaster oder einer iontophoretischen Vorrichtung.

Bevorzugt wird Rotigotin dabei in Pflasterform auf die Haut des Patienten gebracht, wobei der Wirkstoff bevorzugt in einer Matrix aus adhesivem Polymer, z.B. einem selbstklebendem adhesivem Polysiloxan, vorliegt. Beispiele für geeignete transdermale Formulierungen finden sich in WO 99/49852, WO 02/89777, WO 02/89778, WO 04/58247, WO 04/12730, WO 04/12721 oder WO 04/50083. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman, Clinical Neuropharmacol. 24, 2001, 163).

Wird dagegen ein Arzneimittel in Form einer subkutanen oder intramuskularen Depotform gewünscht, kann das Rotigotin beispielsweise als Salzkristall, z.B. als kristallines Hydrochlorid, in einem hydrophobem, wasserfreiem Medium suspendiert und injiziert werden, wie in WO 02/15903 beschrieben oder auch in Form von Mikrokapseln, Mikropartikeln oder Implantaten auf Basis bioabbaubarer Polymere, wie beispielsweise in WO 02/38646 beschrieben, verabreicht werden.

Andere denkbare Formen der Verabreichung von Rotigotin und seinen Prodrugs sind transmukosale Formulierungen, z.B. Sublingual- oder Nasalsprays, rektale Formulierungen oder Aerosole zur pulmonalen Verabreichung.

Geeignete Dosierungen von Rotigotin liegen zwischen 0,05 und 50 mg/Tag, wobei vorzugsweise Tagesdosen zwischen 0,1 und 40 mg und insbesondere zwischen 0,2 und 20 mg/Tag verabreicht werden. Dabei kann die Dosierung einschleichend erfolgen, das heißt, die Behandlung kann gegebenenfalls mit niedrigen Dosierungen beginnen, die dann bis zur Erhaltungsdosis gesteigert werden.

Dem Fachmann ist klar, dass das Dosierungsintervall in Abhängigkeit von der applizierten Menge, der Applikationsart und dem Tagesbedarf des Patienten variieren kann. So kann eine transdermale Applikationsform beispielsweise zur einmal täglichen, dreitägigen oder siebentägigen Verabreichung konzipiert sein, während ein subkutanes oder intramuskuläres Depot Injektionen beispielsweise im Ein-, Zwei- oder Vierwochen-Rhythmus ermöglichen kann.

In der neuroprotektiven Arzneiform können neben Rotigotin noch andere Wirkstoffe vorliegen, die die Progression des dopaminergen Zellverlusts verhindern.

Beispiele hierfür sind antiapoptotisch wirksame Substanzen (Minocycline, FK-506, Cyclosporin A, zVAD) sowie Neurotrophine, wie z.B. der Glial-cell-derived neurotrophic factor (GDNF).

In einem Kombinationspräparat kann die Freisetzung der jeweils eingesetzten Wirkstoffe weitgehend simultan oder aber sequentiell erfolgen. Eine sequentielle Gabe kann beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Ein erfindungsgemäßes Kombinationspräparat umfassend eine Rotigotinformulierung kann alternativ auch als sogenanntes "Kit of parts" beschaffen sein, in dem die zu verabreichenden antiapoptotischen Wirkstoffe in voneinander getrennten Formulierungen vorliegen, die dann gleichzeitig oder zeitlich abgestuft verabreicht werden.

Dem Fachmann ist klar, dass im Kontext der vorliegenden Erfindung verschiedene Darreichungsformen und Applikationsschemata denkbar sind.

### Ausführungsbeispiele:

### 1. Ausführungsbeispiel: Rotigotin-Pflaster

1.8 g Rotigotin (freie Base) werden in 2.4 g Ethanol gelöst und zu 0.4 g Kollidon 90F (gelöst in 1g Ethanol) gegeben. Diese Mischung wird zu einer 74%igen Lösung von Silikonpolymeren (8.9 g BioPSA 7-4201 + 8.9 g BIO-PSA 7-4301 [DowCorning]) in Heptan gegeben. Nach Zugabe von 2.65 g Petrolether wird die Mischung für 1 Stunde bei 700 UpM gerührt um eine homogene Dispersion zu erhalten. Nach Laminierung auf Polyester wurde bei 50°C getrocknet. Das Pflastergewicht betrug schließlich 50 g/cm2.

### 2. Ausführungsbeispiel: Rotigotin-Depotsuspensionen

(a) 1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.
(b) 1188 g der unter (a) hergestellten Lösung wurde in einen Glaslaborreaktor überführt, 12 g Rotigotin HCl zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### 3. Ausführungsbeispiel: Subakutes MPTP-Modell

Zur Intoxikation werden Mäusen 80 mg/kg des Neurotoxins 1-methyl-4-phenyl-1,2,3,6-tetrahydro-pyridine (MPTP) verabreicht (in Portionen von 20 mg/kg in zweistündigen Abständen, Gruppe 3-6 in Abbildung 1 und 2), was dazu führt, dass ca. 50 - 60 % der Neurone der Substantia nigra degenerieren (maximale Degeneration in Gruppe 3 in Abbildung 1 und 2). Rotigotin wird täglich in Dosen zu je 0.3, 1 oder 3 mg/kg über 7 Tage als sogenannte ,Slow-Release-Formulierung' (siehe Ausführungsbeispiel 2) verabreicht (Gruppe 4-6 in Abbildung 1 und 2). Eine Gruppe MPTP-behandelter Tiere (Gruppe 3) erhält Rotigotin-Vehikel-Lösung (siehe Ausführungsbeispiel 2 ohne Rotigotin HCL) und dient als Referenz. Als Kontrollen dienen die Gruppen 1, 2 und 7, wobei Gruppe 1 keinerlei Behandlung erfährt, Gruppe 2 mit den Vehikel-Lösungen für MPTP und Rotigotin behandelt wird und Gruppe 7 ausschließlich Rotigotin erhält. Am 8. Tag werden die Tiere getötet, die Gehirne entnommen und eingefroren. Gefrierschnitte werden mit 100 pm [¹²⁵|] PE2| ([¹²⁵|]-(E)-N(3-iodoprop-2-enyl)-2β-carboxymethyl-3β-(4'-methylphenyl)-nortropane) in Phosphat Puffer, pH 7.4, inkubiert, um die Menge der im Striatum noch vorhandenen Dopamin-Transporter zu markieren, was als Indiz für die Menge funktionierender Nervenendigungen dient. Rotigotin verbessert das Überteben der Neurone und ihrer Nervenendigungen dosisabhängig. Dies ist ein klarer Hinweise für die neuroprotektiven Eigenschaften der Substanz (Abbildung 1 und 2).

### 4. Ausführungsbeispiel: Akutes MPTP-Modell (einschließlich Apoptose)

Zur Intoxikation werden Mäusen 80 mg/kg des Neurotoxins 1-methyl-4-phenyl-1,2,3,6-tetrahydro-pyridine (MPTP) verabreicht (in Portionen von 20 mg/kg in zweistündigen Abständen), was dazu führt, dass ca. 50 - 60 % der Neurone der Substantia nigra degenerieren. Ca 16. Stunden vorher wird Rotigotin in Dosen zu je 0.3, 1. oder 3 mg/kg als sogenannte ,Slow-Release-Formulierung'(siehe Ausführungsbeispiel 2) verabreicht. Diffusions-und Absorptionslatenzen führen dazu, daß Rotigotin dann optimal verfügbar ist, wenn MPTP gegeben wird. Tiere, die nur Vehikellösung (siehe Ausführungsbeispiel 2 ohne Rotigotin HCl) erhalten haben, dienen als Kontrollen. Nach 24 Stunden werden die Tiere getötet und die Gehirne fixiert. Die Gehirnschnitte werden mit FluoroJade zur Identifzierung degenerierender Zellen gefärbt. Die immunhistochemische Markierung der Tyrosin-Hydroxylase dient der Identifizierung dopaminerger Neurone. Die Färbung der Tyrosin-Hydroxylase ergibt keine Unterschiede zwischen behandelten und unbehandelten Tieren; die Färbung mit FluoroJade zeigt eine große Zahl degenerierender Neurone; die Neurone sind allerdings noch nicht vollständig entfernt (was den fehlenden Unterschied der Tyrosin-Hydroxylase-Färbung erklärt); dies legt nahe, dass der Zelluntergang apoptotisch verläuft und zum Messzeitpunkt noch nicht abgeschlossen ist (die apoptotischen Zellen sind noch nicht vollständing aufgelöst bzw. phagozytiert). Die Anzahl der degenerierenden Neurone ist um ca. 50 % geringer nach Applikation von Rotigotin, was die neuroprotektive Eigenschaft der Substanz weiter belegt (Tabelle 2).

### 5. Ausführungsbeispiel: In vitro Umsetzung eines Prodrugs in die Wirksubstanz

Aus Leberzellhomogenaten von Mensch, Affe, Hund, Ratte oder Mouse wird durch differentielle Zentrifugation die Mikrosomenfraktion erhalten, die die wesentlichen metabolisierenden Enzyme enthält; alternative kann auch die zytoplasmatische Fraktion gewonnen werden. Die subzelluläre Fraktion wird mit einem Puffer so suspendiert dass eine Lösung mit einem definierten Proteingehalt erhalten wird. Nach Zufügen von 1 µM des zu testenden Prodrugs erfolgt die Inkubation bei 37 °C für 60 min. Anschließend wird Rotigotin mittels HPLC/UV oder auch mittels HPLC/MS quantifiziert und zur eingesetzten Menge in Beziehung gesetzt. Für detailliertere Analysen werden Konzentrations- oder Zeitreihen untersucht.

## Patentansprüche

1. Verwendung von Rotigotin, seiner Salze und Prodrugs zur Herstellung eines Arzneimittels zur Prävention und/oder Behandlung des Parkinson-Plus-Syndroms,
wobei die Prodrugs aus der Gruppe bestehend aus Alkylcarbonylestern -C(O)-Alkyl; Cycloalkylcarbonylestern -C(O)-Cycloalkyl; Arylcarbonylestern -C(O)-Aryl; Carbonaten -C(O)-O-R; Carbamaten -C(O)-NRR1, -C(O)-NHR1 oder -C(O)-NH₂; Acetalen -CH(OR)R1; Ketalen -C(OR)R1R2; Acyloxyalkylethern -CHR-O-C(O)-R1 oder -CH₂-O-C(O)-R1; Phosphaten -P(O₂H)OR; Phosphonaten -P(O₂H)R; Sulfaten -S(O)₂OR; Sulfonaten -S(O)₂R; Thiocarbonylestern -C(=S)-R; Oxothiocarbonylestern -C(=S)-O-R; Thiocarbamaten -C(=S)-NRR1, -C(=S)-NHR1 oder -C(=S)-NH₂; und Ethern -R der phenolischen Hydroxygruppe von Rotigotin ausgewählt sind, und
worin R, R1 und R2 jeweils unabhängig aus Wasserstoff, Alkyl, Cycloalkyl oder Aryl ausgewählt sind.

2. Verwendung nach Anspruch 1, wobei das Parkinson-Plus-Syndrom ausgewählt ist aus der Gruppe der Multisystematrophien, der progressiven supranukleären Blickparese, der kortikobasalen Degeneration und der diffusen Lewy-Körperchen-Demenz.

3. Verwendung nach einem der vorhergehenden Ansprüche, wobei das Parkinson-Plus-Syndrom durch das fehlende Ansprechen von L-Dopa **gekennzeichnet** ist.

4. Verwendung nach einem der vorhergehenden Ansprüche, wobei das Arzneimittel zur parenteralen, transdermalen oder transmukosalen Verabreichung ausgebildet ist.

5. Verwendung nach einem der vorhergehenden Ansprüche, wobei das Rotigotin in einer Dosierung von 0,05 - 50 mg pro Tag zu verabreichen ist.

6. Verwendung nach einem der vorhergehenden Ansprüche, wobei das Prodrug ein Alkylcarbonylester mit bis zu 6 Kohlenstoffatomen ist.

7. Verwendung nach einem der vorhergehenden Ansprüche, wobei das Arzneimittel zur transdermalen Verabreichung ausgebildet ist.

8. Verwendung nach Anspruch 7, wobei die Darreichungsform aus Salbe, Paste, Spray, Folie, Pflaster oder einer iontophoretischen Vorrichtung ausgewählt ist.

9. Verwendung nach Anspruch 7, wobei die Darreichungsform ein Pflaster ist.

## Claims

1. Use of rotigotine, its salts and prodrugs for producing a medicament for the prevention and/or treatment of Parkinson's plus syndrome,
wherein the prodrugs are selected from the group consisting of alkylcarbonyl esters -C(O)-alkyl; cycloalkylcarbonyl esters -C(O)-cycloalkyl; arylcarbonyl esters -C(O)-aryl; carbonates -C(O)-O-R; carbamates -C(O)-NRR1, -C(O)-NHR1 or -C(O)-NH₂; acetals -CH(OR)R1; ketals -C(OR)R1R2; acyloxyalkyl ethers -CHR-O-C(O)-R1 or -CH₂-O-C(O)-R1; phosphates -P(O₂H)OR; phosphonates -P(O₂H)R sulphates -S(O)₂OR; sulphonates -S(O)₂R; thiocarbonyl esters -C(=S)-R; oxothiocarbonyl esters -C(=S)-O-R; thiocarbamates -C(=S)-NRR1, -C(=S)-NHR1 or -C(=S)-NH₂; and ethers -R of the phenolic hydroxy group of rotigotine, and
wherein R, R1 and R2 are selected in each case independently from hydrogen, alkyl, cycloalkyl or aryl.

2. Use according to claim 1, wherein the Parkinson's plus syndrome is selected from the group of multi-system atrophies, progressive supranuclear palsy, corticobasal degeneration and diffuse Lewy body dementia.

3. Use according to one of the preceding claims, wherein the Parkinson's plus syndrome is **characterised by** the lack of response by L-dopa.

4. Use according to one of the preceding claims, wherein the medicament is designed for parenteral, transdermal or transmucosal administration.

5. Use according to one of the preceding claims, wherein the rotigotine is to be administered in a dose of 0.05 - 50 mg per day.

6. Use according to one of the preceding claims, wherein the prodrug is an alkylcarbonyl ester having up to 6 carbon atoms.

7. Use according to one of the preceding claims, wherein the medicament is designed for transdermal administration.

8. Use according to claim 7, wherein the form of administration is selected from ointment, paste, spray, film, plaster or an iontophoretic device.

9. Use according to claim 7, wherein the form of administration is a plaster.

## Revendications

1. Utilisation de la rotigotine, de ses sels et de ses promédicaments, pour la fabrication d'un médicament destiné à la prévention et/ou au traitement du syndrome "Parkinson plus",
dans laquelle les promédicaments sont choisis dans le groupe comprenant les alkylcarbonylesters -C(O)-alkyle ; les cycloalkylcarbonylesters -C(O)-cycloalkyle ; les arylcarbonylesters -C(O)-aryle ; les carbonates -C(O)-O-R ; les carbamates -C(O)-NRR1, -C(O)-NHR1 ou -C(O)-NH₂ ; les acétals -CH(OR)R1 ; les cétals -C(OR)R1R2 ; les acyloxyalkyléthers -CHR-O-C(O)-R1 ou -CH₂-O-C(O)-R1 ; les phosphates -P(O₂H)OR ; les phosphonates -P(O₂H)R ; les sulfates -S(O)₂OR ; les sulfonates -S(O)₂R; les thiocarbonylesters -C(=S)-R ; les oxothiocarbonylesters -C(=S)-O-R ; les thiocarbamates -C(=S)-NRR1, -C(=S)-NHR1 ou -C(=S)-NH₂ ; et les éthers -R du groupe hydroxy phénolique de la rotigotine, et
dans laquelle R, R1 et R2 sont choisis chacun indépendamment parmi hydrogène, alkyle, cycloalkyle ou aryle.

2. Utilisation selon la revendication 1, dans laquelle le syndrome "Parkinson plus" est choisi parmi les atrophies systématisées multiples, les paralysies supranucléaires progressives, la dégénérescence corticobasale et la démence à corps de Loewy diffus.

3. Utilisation selon l'une quelconque des revendications précédentes, dans laquelle le syndrome "Parkinson plus" est **caractérisé par** l'absence de réponse à la L-dopa.

4. Utilisation selon l'une quelconque des revendications précédentes, dans laquelle le médicament est conçu pour une administration parentérale, transdermique ou transmuqueuse.

5. Utilisation selon l'une quelconque des revendications précédentes, dans laquelle la rotigotine doit être administrée à une dose de 0,05 à 50 mg par jour.

6. Utilisation selon l'une quelconque des revendications précédentes, dans laquelle le promédicament est un alkylcarbonylester comportant jusqu'à 6 atomes de carbone.

7. Utilisation selon l'une quelconque des revendications précédentes, dans laquelle le médicament est conçu en vue d'une administration transdermique.

8. Utilisation selon la revendication 7, dans laquelle la forme d'administration est choisie parmi une pommade, une pâte, une pulvérisation, un film, un dispositif transdermique ou un dispositif d'ionophorèse.

9. Utilisation selon la revendication 7, dans laquelle la forme d'administration est un dispositif transdermique.
